# EUROPEAN PATENT SPECIFICATION

(11) **EP 0 723 427 B1**
(45) Date of publication and mention of the grant of the patent: **06.08.1997**
(21) Application number: 94928399.8
(22) Date of filing: 29.09.1994
(51) Int. Cl.: A61B 17/56

(54) **GAUGE FOR THE MEDULLARY CAVITY**
MESSGERÄT FÜR MARKKANAL
JAUGE DE CAVITE MEDULLAIRE

(30) Priority: 12.10.1993 IT VR930074
(43) Date of publication of application: 31.07.1996
(73) Proprietor: ORTHOFIX S.r.l., 37012 Bussolengo (VR) (IT)
(72) Inventor: FACCIOLI, Giovanni, I-46040 Monzambano (IT); ROSSI, Stefano, I-37124 Verona (IT); PENNIG, Dietmar, D-4400 Münster (DE)
(74) Representative: Thomson, Paul Anthony
(86) International application number: EP9403251
(87) International publication number: WO9510237

(56) References cited:
- WO-A-93/15669
- US-A- 3 763 855
- US-A- 5 013 318
- US-A- 5 171 248
- US-A- 5 197 465

## Description

This invention relates to a gauge for the medullary cavity which can be removably inserted into the medullary cavity of a bone.

Before implanting a medullary pin the bone which has to be operated on is normally bored out to assist insertion of the pin. During this stage it is extremely important to avoid forcing the pin because sudden stresses can seriously damage the bone. In many cases it is also difficult to determine the exact length of the pin, and therefore it very frequently happens that a pin which has already been inserted must be removed in order to be replaced by another of the correct length.

It frequently also occurs that the pin does not perfectly fit the bored out medullary cavity so it has to be removed and further boring is required. This situation is made more complicated by the fact that the guide wire or pin which was used for the initial drilling operation has been removed.

In patent application PCT no. DE/93/00134 there is described a gauge in the form of a hollow rod which has external shape and dimensions corresponding to a medullary pin, which has as its proximal end a radial handle and at its distal end an opening for inserting a wire or drilling bit. In the vicinity of the distal end there are provided grooves placed in diametrically opposite positions on the front and rear part of the rod which are used to determine in the bone the position for the pin anchorage bolt holes using X-rays. Towards the proximal end, on the outer part of the rod, there is provided a measuring scale to check and compare the length of medullary pins of standard length, by means of which the length of the drilled hole can be determined and the size of the pin which has to be inserted can be determined with accuracy. In this way, after the rod has been inserted into the medullary cavity, its length can be detected so as to determine the accurate length of the pin which has to be inserted. Also, after the position of one or more grooves on the inserted gauge has been determined, it is possible to establish where the transverse holes for one or more bolts to anchor the medullary pin to the bone must be drilled.

This device assists the work of the surgeon, but it can be improved in some respects. First of all it does not make it possible to establish whether the bored out cavity has a sufficient diameter for free insertion of the pin in the cavity. In addition to this, in order to determine the length of the bored out cavity a reading has to be made on the scale of the gauge when this is inserted in the bone, after which the value read has to be remembered by the surgeon, with the possibility of confusion and error. Finally, a gauge of corresponding shape having an integral handle has to be provided for every diameter and shape of medullary cavity.

The object of this invention is to provide a gauge far intramedullary cavities which overcomes the abovementioned disadvantages.

According to the present invention there is provided a gauge for medullary cavities, comprising a hollow rod having approximately the shape and dimensions of a medullary pin, the said rod having in the vicinity of its distal end one or more circumferential grooves to locate the holes for the transverse fixing bolts for the pin, and in the vicinity of its proximal end a scale which is visible externally for measuring the length of the cavity this rod being provided with a substantially radial handle, characterized in that the said handle is slidably mounted on the said rod along at least part of its length.

Some preferred embodiments are described below by way of non-limitative examples with reference to the appended drawings in which:
Figure 1 is a side view of the gauge according to the invention in a first embodiment, positioned within the medullary cavity of a bone,
Figure 2 is a perspective view of the gauge in Figure 1,
Figure 3 is a sectional view of a detail of the gauge in Figure 2 taken along a section plane II-II,
Figure 4 is a view similar to that in Figure 3 with a slightly modified form of the said detail.

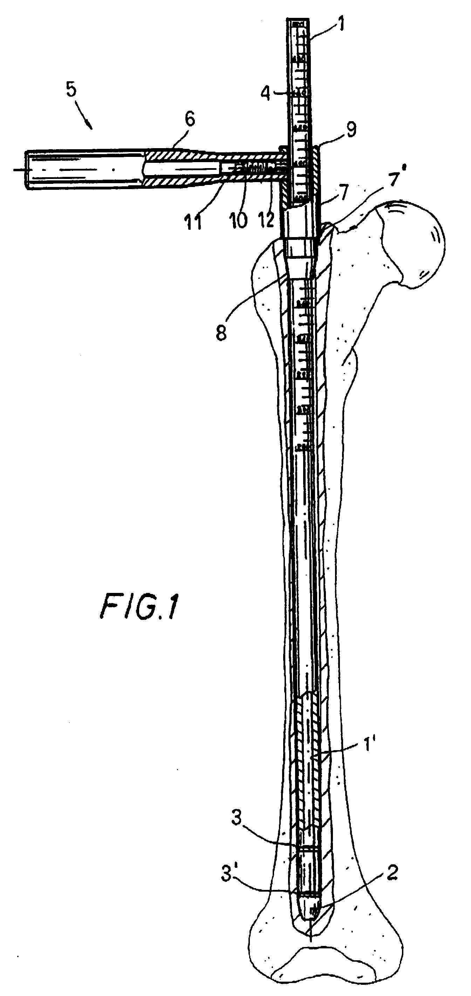

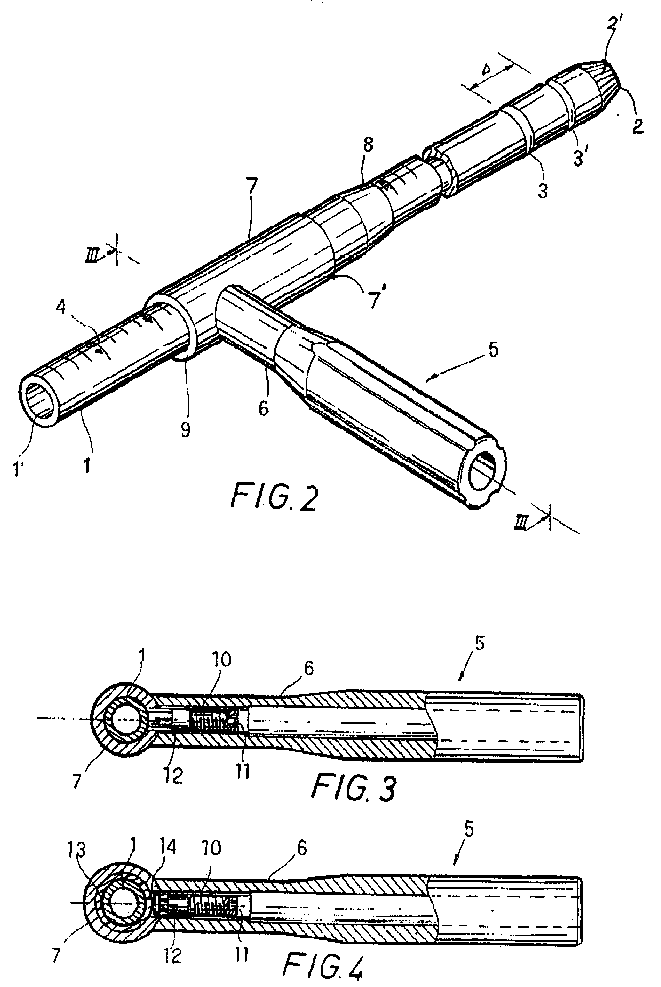

Figure 1 shows a gauge for medullary cavities according to the invention which comprises a hollow rod 1 with a through hole 1' for a guide wire. Rod 1 is substantially cylindrical with an external diameter equal to the diameter of the distal end of a standard medullary pin and its distal end 2 is tapered and rounded to assist insertion into the medullary cavity. As may be seen in Figure 2, the tapering distal end 2 of the rod 1 can be provided with grooves 2' which form small cutting edges. By means of such cutting edges 2' the surgeon can remove any minor impediments and irregularities in the cavity using the gauge as a boring device, without having to remove it and again inserting the drilling tool into the cavity.

In the vicinity of the distal end 2 there are provided two circumferential grooves 3, 3', which may be complete or incomplete, spaced longitudinally at a distance Δ such as to locate the centres of the transverse holes for a medullary pin, or a series of pins of different lengths which can be used with the gauge according to the invention.

In the vicinity of the proximal end there is incised a measurement scale 4 which extends some distance from the distal end 2 of rod 1.

On rod 1 there is mounted a slidable handle 5 comprising a radial tubular body 6 with one end fixed to a sleeve 7 which has an internal diameter slightly greater than the external diameter of rod 1. Sleeve 7 may be mounted on the rod 1 and may slide longitudinally along it. Such sleeve 7 has an external shape conforming a jig, not illustrated in the figures, that can be used by the surgeon to support the pin during implantation thereof in the medullary cavity. In particular sleeve 7 has towards the distal end 2 of rod 1 a first portion of reduced diameter providing a circular stepped edge 7' corresponding to the distal end of the implantation jig, and a second tapered portion with a frustoconical end 8. The taper angle of frustoconical portion 8 is such as to permit it to be inserted down to the very end of the hole which has been bored in the medullary cavity in order to check whether the bore is sufficient or must be remade. The rear end of sleeve 7 has a circumferential edge 9 which can be aligned with reference marks on scale 4 to measure the length of the bored hole.

A cylindrical member 10 is inserted within tubular body 6 in the vicinity of sleeve 7 and is threaded externally to engage a corresponding internal thread in tubular body 6. The flat end surface of cylindrical member 10 which faces outward has centrally an inset hexagon 11 for the insertion of a tool having a hexagonal cross-section. The opposite side of the cylindrical member acts against a cylindrical peg 12 which is slidably mounted within the tubular body and acts against the rod with its outer end to lock it with respect to the handle by screwing up cylindrical member 10.

In the modified embodiment illustrated in Figure 3, the distal end of handle 7 is mounted on rod 1 with an interposed sleeve 13 of deformable plastics material. Cylindrical peg 12 has a rigid head at its inner end which acts on sleeve 13 to deform it and immobilise handle 5 on rod 1.

In use, when hollow rod 1 has been inserted into the medullary cavity bored with the help of a guide wire, it is possible to check whether the gauge enters freely or whether it jams because the diameter of the medullary cavity is insufficient. In the latter case rod 1 must be removed and the cavity has to be rebored to provide a larger diameter.

Once rod 1 has been fully inserted and grooves 3, 3' has been checked by means of X-rays to verify that they are positioned beyond the fracture, cylindrical peg 12 is unlocked by unscrewing cylindrical member 10 and the handle is released from rod 1. Handle 5 is then reinserted into the proximal part of the medullary cavity until stepped edge 7' of sleeve 7 is brought into abutment agains the proximal end of the bored medullary cavity. At this point the length of the bored cavity can be read by comparing the rear edge 9 of sleeve 7 with scale 4 in order to choose a medullary pin of appropriate length.

## Claims

1. A gauge for medullary cavities, comprising a hollow rod (1) having approximately the shape and dimensions of a medullary pin, the said rod (1) having in the vicinity of its distal end (2) one or more circumferential grooves (3, 3') to locate the holes for the transverse fixing bolts for the pin, and in the vicinity of its proximal end a scale (4) which is visible externally for measuring the length of the cavity, this rod being provided with a substantially radial handle (5), characterised in that the said handle (5) is slidably mounted on the said rod (1) along at least part of its length.

2. A gauge for medullary cavities according to claim 1, in which the said handle (5) comprises a tubular body (6) having one end fixed to a sleeve (7) slidably mounted upon the said rod (1).

3. A gauge for medullary cavities according to claim 2, in which the sleeve (7) has its end portion (8) facing the distal end of the rod of a frustoconical shape with a predetermined taper angle, which can be inserted at least partly into the medullary cavity to check its longitudinal position.

4. A gauge for medullary cavities according to claim 3, in which the end (9) of the said sleeve opposite the frustoconical end has a circumferential edge (9) which can be aligned with reference marks on the said scale (4) to read the length of the medullary cavity.

5. A gauge for medullary cavities according to claim 4, in which the said tubular member (6) houses slidably within itself adjustable friction means (10, 12) capable of immobilising the said handle (5) in a given longitudinal position on the said rod (1).

6. A gauge for medullary cavities according to claim 5, in which the said adjustable friction means (10, 12) comprise an external thread which can be coupled with an internal thread in the said tubular member (6), means (11) for operating the said friction means (10, 12) which are accessible from the exterior being provided.

7. A gauge for medullary cavities according to claim 5, in which a sleeve (13) of material which can be deformed by means of the said adjustable means (12, 14) to immobilise the handle (5) is placed between the said sleeve (7) and the said rod (1).

8. A gauge for medullary cavities according to claim 1, in which the distal end (2) of the said hollow rod (1) has shallow grooves (2') which act as cutters.

## Patentansprüche

1. Eine Lehre für Markhöhlen, die aus einer Hohlstange (1) mit der ungefähren Form und den ungefähren Abmessungen eines Marknagels besteht, wobei die besagte Stange (1) in der Nähe ihres distalen Endes (2) mit einer oder mehreren Umfangsrillen (3, 3'), um die Löcher für die Querbefestigungsschrauben für den Nagel zu bestimmen, und in der Nähe ihres proximalen Endes mit einer von außen sichtbaren Skala (4) zum Messen der Länge der Höhle versehen ist, und wobei diese Stange einen im wesentlichen radialen Griff (5) aufweist; dadurch gekennzeichnet, daß der besagte Griff (5) auf der besagten Stange (1) entlang mindestens eines Teils ihrer Länge verschiebbar angeordnet ist.

2. Eine Lehre für Markhöhlen nach Anspruch 1, in der der besagte Griff (1) aus einem röhrenförmigen Körper (6) besteht, der ein Ende aufweist, das an einer verschiebbar auf der besagten Stange (1 ) angebrachten Hülse (7) befestigt ist.

3. Eine Lehre für Markhöhlen nach Anspruch 2, in der der Endabschnitt (8) der Hülse (7) dem distalen Ende der Stange von stumpfkegeliger Form mit einem vorherbestimmten Kegelwinkel zugekehrt ist, der zumindest teilweise in die Markhöhle eingeführt werden kann, um ihre Längsposition zu kontrollieren.

4. Eine Lehre für Markhöhlen nach Anspruch 3, in der das Ende (9) der besagten Hülse gegenüber dem stumpfkegeligen Ende eine Umfangskante (9) hat, die mit Bezugsmarkierungen auf der besagten Skala (4) ausgerichtet werden kann, um die Länge der Markhöhle abzulesen.

5. Eine Lehre für Markhöhlen nach Anspruch 4, in der das besagte röhrenförmige Teil (6) verschiebbar in seinem Innern verstellbare Friktionshilfsmittel (10, 12) aufweist, die in der Lage sind, den besagten Griff (5) in einer gegebenen Längsposition auf der besagten Stange (1) ruhigzustellen.

6. Eine Lehre für Markhöhlen nach Anspruch 5, in der die besagten verstellbaren Friktionsmittel (10, 12) ein Außengewinde umfassen, das mit einem Innengewinde in dem besagten röhrenförmigen Teil (6) verbunden werden kann, wobei ein Hilfsmittel (11) zum Betätigen der besagten Friktionshilfsmittel (10, 12), die von außen zugänglich sind, vorgesehen ist.

7. Eine Lehre für Markhöhlen nach Anspruch 5, in der eine Hülse (13) aus einem Material, das mittels der besagten verstellbaren Hilfsmittel (12, 14) vertormt werden kann, um den Griff (5) ruhigzustellen, zwischen der besagten Hülse (7) und der besagten Stange (1) positioniert ist.

8. Eine Lehre für Markhöhlen nach Anspruch 1, in der das distale Ende (2) der besagten Hohlstange (1) flache Rillen (2') aufweist, die als Schneidwerkzeuge wirken.

## Revendications

1. Jauge de cavité médullaire, comprenant une tige creuse (1) dont la forme et les dimensions sont approximativement égales à celles d'une broche médullaire, ladite tige (1) ayant à proximité de son extrémité distale (2) une ou plusieurs rainures circonférentielles (3, 3') destinées à localiser les trous pour les boulons de fixation transversaux de la broche, et à proximité de son extrémité proximale une échelle (4) visible à l'extérieur pour mesurer la longueur de la cavité, cette tige étant pourvue d'une poignée (5) sensiblement radiale, caractérisée en ce que ladite poignée (5) peut coulisser sur ladite tige (1) au moins sur une partie de sa longueur.

2. Jauge de cavité médullaire selon la revendication 1, caractérisée en ce que ladite poignée (5) comprend un corps tubulaire (6) avec une extrémité fixée à un manchon (7) monté de façon coulissante sur ladite tige (1).

3. Jauge de cavité médullaire selon la revendication 2, caractérisée en ce que l'extrémité (8) du manchon (7) se trouve face à l'extrémité distale de la tige en forme de tronc de cône ayant un angle de cône prédéterminé, qui peut être insérée au moins partiellement dans la cavité médullaire pour vérifier sa position longitudinale.

4. Jauge de cavité médullaire selon la revendication 3, caractérisée en ce que l'extrémité (9) du dudit manchon opposée à l'extrémité tronconique possède une arête circonférentielle (9) qui peut être alignée sur des marquages de référence de ladite échelle (4) pour mesurer la longueur de la cavité médullaire.

5. Jauge de cavité médullaire selon la revendication 4, caractérisée en ce que l'élément tubulaire (6) abrite d'une manière coulissante par rapport à lui-même des moyens de friction réglables (10, 12) pouvant immobiliser ladite poignée (5) dans une position longitudinale donnée sur ladite tige (1).

6. Jauge de cavité médullaire selon la revendication 5, caractérisée en ce que lesdits moyens de friction réglables (10, 12) comprennent un filet extérieur qui peut être couplé avec un filet intérieur dans ledit élément tubulaire (6), des moyens (11) accessibles de l'extérieur étant prévus pour faire fonctionner lesdits moyens de friction (10, 12).

7. Jauge de cavité médullaire selon la revendication 5, caractérisée en ce qu'un manchon (13) fait d'une matière qui peut être déformée au moyen desdits moyens réglables (12, 14) pour immobiliser la poignée (5) est disposé entre ledit manchon (7) et ladite tige (1).

8. Jauge de cavité médullaire selon la revendication 1, caractérisée en ce que l'extrémité distale (2) de ladite tige creuse (1) possède des rainures peu profondes (2') qui servent d'outils de coupe.
